# EUROPEAN PATENT APPLICATION

(11) **EP 3 693 063 A1**
(43) Date of publication of application: **12.08.2020**
(21) Application number: 19305140.6
(22) Date of filing: 06.02.2019
(51) Int. Cl.: A61P 35/00, A61K 39/395, C07K 16/40

(54) **METHODS AND COMPOSITIONS FOR TREATING CANCER**

(71) Applicant: Diaccurate, 75008 Paris (FR)
(72) Inventor: THEZE, Jacques, 75007 PARIS (FR); TAMARIT, Blanche, 75014 PARIS (FR); POULETTY, Philippe, 75005 PARIS (FR)
(74) Representative: Cabinet Becker et Associés

(57) **Abstract**

The present invention relates to compositions and methods for treating cancers in a subject in need thereof. The invention more particularly relates to methods of treating cancers by inhibiting PLA2GIB in a subject in need thereof.

SEQUENCE LISTING

<110>  DIACCURATE

<120>  METHODS AND COMPOSITIONS FOR TREATING CANCER

<130>  B2952

<160>  34

<170>  PatentIn version 3.5

<210>  1
<211>  148
<212>  PRT
<213>  Homo sapiens

<400>  1

Met Lys Leu Leu Val Leu Ala Val Leu Leu Thr Val Ala Ala Ala Asp
1               5                   10                  15

Ser Gly Ile Ser Pro Arg Ala Val Trp Gln Phe Arg Lys Met Ile Lys
            20                  25                  30

Cys Val Ile Pro Gly Ser Asp Pro Phe Leu Glu Tyr Asn Asn Tyr Gly
        35                  40                  45

Cys Tyr Cys Gly Leu Gly Gly Ser Gly Thr Pro Val Asp Glu Leu Asp
    50                  55                  60

Lys Cys Cys Gln Thr His Asp Asn Cys Tyr Asp Gln Ala Lys Lys Leu
65                  70                  75                  80

Asp Ser Cys Lys Phe Leu Leu Asp Asn Pro Tyr Thr His Thr Tyr Ser
                85                  90                  95

Tyr Ser Cys Ser Gly Ser Ala Ile Thr Cys Ser Ser Lys Asn Lys Glu
            100                 105                 110

Cys Glu Ala Phe Ile Cys Asn Cys Asp Arg Asn Ala Ala Ile Cys Phe
        115                 120                 125

Ser Lys Ala Pro Tyr Asn Lys Ala His Lys Asn Leu Asp Thr Lys Lys
    130                 135                 140

Tyr Cys Gln Ser
145

<210>  2
<211>  126
<212>  PRT
<213>  Homo sapiens

<400>  2

Ala Val Trp Gln Phe Arg Lys Met Ile Lys Cys Val Ile Pro Gly Ser
1               5                   10                  15

Asp Pro Phe Leu Glu Tyr Asn Asn Tyr Gly Cys Tyr Cys Gly Leu Gly
            20                  25                  30

Gly Ser Gly Thr Pro Val Asp Glu Leu Asp Lys Cys Cys Gln Thr His
        35                  40                  45

Asp Asn Cys Tyr Asp Gln Ala Lys Lys Leu Asp Ser Cys Lys Phe Leu
    50                  55                  60

Leu Asp Asn Pro Tyr Thr His Thr Tyr Ser Tyr Ser Cys Ser Gly Ser
65                  70                  75                  80

Ala Ile Thr Cys Ser Ser Lys Asn Lys Glu Cys Glu Ala Phe Ile Cys
                85                  90                  95

Asn Cys Asp Arg Asn Ala Ala Ile Cys Phe Ser Lys Ala Pro Tyr Asn
            100                 105                 110

Lys Ala His Lys Asn Leu Asp Thr Lys Lys Tyr Cys Gln Ser
        115                 120                 125

<210>  3
<211>  117
<212>  PRT
<213>  Artificial Sequence

<220>
<223>  14G9 VL

<400>  3

Asp Ile Val Met Thr Gln Ser His Lys Leu Met Ser Thr Ser Val Gly
1               5                   10                  15

Asp Arg Val Ser Ile Thr Cys Lys Ala Ser Gln Asp Val Ser Thr Ala
            20                  25                  30

Val Ala Trp Tyr Gln Glu Lys Pro Gly Gln Ser Pro Lys Leu Leu Ile
        35                  40                  45

Tyr Trp Ala Ser Thr Arg His Thr Gly Val Pro Asn Arg Phe Thr Gly
    50                  55                  60

Ser Arg Ser Gly Thr Asp Tyr Thr Leu Thr Ile Ser Ser Val Gln Ala
65                  70                  75                  80

Glu Asp Leu Ala Leu Tyr Tyr Cys Gln Gln Asp Tyr Ser Thr Pro Pro
                85                  90                  95

Thr Phe Gly Ala Gly Thr Lys Leu Glu Leu Lys Arg Ala Asp Ala Ala
            100                 105                 110

Pro Thr Val Ser Ile
        115

<210>  4
<211>  130
<212>  PRT
<213>  Artificial Sequence

<220>
<223>  14G9 VH

<400>  4

Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Asn Tyr
            20                  25                  30

Trp Ile His Trp Val Lys Arg Arg Pro Gly Gln Gly Leu Glu Trp Ile
        35                  40                  45

Gly Asn Ile Asp Pro Ser Asp Thr Arg Thr His Tyr Asn Gln Lys Phe
    50                  55                  60

Lys Asp Lys Ala Thr Leu Ala Val Asp Lys Ser Ser Ser Thr Ala Tyr
65                  70                  75                  80

Met Gln Leu Ser Ser Leu Thr Ser Glu Asp Ser Ala Val Phe Tyr Cys
                85                  90                  95

Ala Arg Gln Thr Leu Tyr Tyr Glu Ala Leu Asp Tyr Trp Gly Gln Gly
            100                 105                 110

Thr Thr Leu Thr Val Ser Ser Ala Lys Thr Thr Pro Pro Ser Val Tyr
        115                 120                 125

Pro Leu
    130

<210>  5
<211>  214
<212>  PRT

<213>  Artificial Sequence

<220>
<223>  2B L

<400>  5

Glu Ile Val Met Thr Gln Ser Pro Ala Thr Leu Ser Val Ser Pro Gly
1               5                   10                  15

Glu Arg Ala Thr Leu Ser Cys Lys Ala Ser Gln Asp Val Ser Thr Ala
            20                  25                  30

Val Ala Trp Tyr Gln Gln Lys Pro Gly Gln Ala Pro Arg Leu Leu Ile
        35                  40                  45

Tyr Trp Ala Ser Thr Arg His Thr Gly Ile Pro Ala Arg Phe Ser Gly
    50                  55                  60

Ser Arg Ser Gly Thr Glu Tyr Thr Leu Thr Ile Ser Ser Leu Gln Ser
65                  70                  75                  80

Glu Asp Phe Ala Val Tyr Tyr Cys Gln Gln Asp Tyr Ser Thr Pro Pro
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  6
<211>  449
<212>  PRT
<213>  Artificial Sequence

<220>
<223>  2B H

<400>  6

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Trp Ile His Trp Val Arg Gln Ala Pro Gly Gln Gly Leu Glu Trp Ile
        35                  40                  45

Gly Asn Ile Asp Pro Ser Asp Thr Arg Thr His Tyr Asn Gln Lys Phe
    50                  55                  60

Lys Asp Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gln Thr Leu Tyr Tyr Glu Ala Leu Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys

210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  7
<211>  449
<212>  PRT
<213>  Artificial Sequence

<220>
<223>  2B1 H

<400>  7

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Trp Ile His Trp Val Arg Gln Ala Pro Gly Gln Gly Leu Glu Trp Ile
        35                  40                  45

Gly Asn Ile Asp Pro Ser Asp Thr Arg Thr His Tyr Asn Gln Lys Phe
    50                  55                  60

Lys Asp Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gln Thr Leu Tyr Tyr Glu Ala Leu Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys

210                 215                 220

Thr His Thr Cys Pro Pro Cys Pro Ala Pro Glu Ala Glu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  8
<211>  449
<212>  PRT
<213>  Artificial Sequence

<220>
<223>  2B2 H

<400>  8

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Trp Ile His Trp Val Arg Gln Ala Pro Gly Gln Gly Leu Glu Trp Ile
        35                  40                  45

Gly Asn Ile Asp Pro Ser Asp Thr Arg Thr His Tyr Asn Gln Lys Phe
    50                  55                  60

Lys Asp Arg Ala Thr Leu Thr Val Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gln Thr Leu Tyr Tyr Glu Ala Leu Asp Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Arg Val Glu Pro Lys Ser Cys Asp Lys

210                 215                 220

Thr His Thr Cys Pro Pro Cys Pro Ala Pro Glu Ala Glu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Ser Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  9
<211>  726
<212>  DNA
<213>  Artificial Sequence

<220>
<223>  2B L

<400>  9
gcggccgcca tgaattttgg actgaggctg attttcctgg tgctgaccct gaaaggcgtc      60

cagtgtgaga tcgtgatgac gcagtcgccg gcgacgctga gcgtgagtcc gggggagcgg     120

gcgacgctga gctgcaaggc gtcgcaggac gtgagcacgg ccgtcgcgtg gtatcaacag     180

aagccggggc aagcgccgcg gctgctgatc tactgggcga gcacgcggca cacgggcatc     240

ccggcgcgat tctcggggag ccggagcggg acggagtaca cgctgacgat ctcgtcgctg     300

caaagcgagg acttcgccgt ctactactgc cagcaggact actcgacgcc gccgacgttc     360

ggggggggta ccaaggtcga gatcaaacgt acggtcgcgg cgccttctgt gttcattttc      420

cccccatctg atgaacagct gaaatctggc actgcttctg tggtctgtct gctgaacaac     480

ttctacccta gagaggccaa agtccagtgg aaagtggaca atgctctgca gagtgggaat     540

tcccaggaat ctgtcactga gcaggactct aaggatagca catactccct gtcctctact     600

ctgacactga gcaaggctga ttacgagaaa cacaaagtgt acgcctgtga agtcacacat     660

caggggctgt ctagtcctgt gaccaaatcc ttcaataggg gagagtgctg atagtaaaag     720

ctttga                                                                726

<210>  10
<211>  1431
<212>  DNA
<213>  Artificial Sequence

<220>
<223>  2B H

<400>  10
gcggccgcca tgaattttgg actgaggctg attttcctgg tgctgaccct gaaaggcgtc      60

cagtgtcaag tccagctcgt ccagagcggg gcggaagtca agaagccggg ggcgagcgtg     120

aaagtctcgt gcaaggcgag cggatatacg ttcacgaact actggattca ctgggtccgg     180

caagcgccgg ggcaggggct ggagtggatc gggaacatcg acccgtcgga cacgcggacg     240

cactacaacc agaagttcaa ggaccgggcg acgctgaccg tcgacaagag cacgagcacg     300

gcgtacatgg agctgtcgag cctgcggagc gaggacacgg ccgtctacta ctgcgcgcgg     360

cagacgctgt actacgaggc gctggactac tgggggcagg ggacgctcgt cacggtctcg     420

agcgctagca caaagggccc tagtgtgttt cctctggctc cctcttccaa atccacttct     480

ggtggcactg ctgctctggg atgcctggtg aaggattact ttcctgaacc tgtgactgtc     540

tcatggaact ctggtgctct gacttctggt gtccacactt tccctgctgt gctgcagtct 600

agtggactgt actctctgtc atctgtggtc actgtgccct cttcatctct gggaacccag 660

acctacattt gtaatgtgaa ccacaaacca tccaacacta aagtggacaa aagagtggaa 720

cccaaatcct gtgacaaaac ccacacctgc ccaccttgtc ctgcccctga actgctggga 780

ggaccttctg tgtttctgtt cccccccaaa ccaaaggata ccctgatgat ctctagaacc 840

cctgaggtga catgtgtggt ggtggatgtg tctcatgagg accctgaggt caaattcaac 900

tggtacgtgg atggagtgga agtccacaat gccaaaacca agcctagaga ggaacagtac 960

aattcaacct acagagtggt cagtgtgctg actgtgctgc atcaggattg gctgaatggc 1020

aaggaataca agtgtaaagt ctcaaacaag gccctgcctg ctccaattga gaaaacaatc 1080

tcaaaggcca agggacagcc tagggaaccc caggtctaca ccctgccacc ttcaagagag 1140

gaaatgacca aaaaccaggt gtccctgaca tgcctggtca aaggcttcta cccttctgac 1200

attgctgtgg agtgggagtc aaatggacag cctgagaaca actacaaaac aaccccccct 1260

gtgctggatt ctgatggctc tttctttctg tactccaaac tgactgtgga caagtctaga 1320

tggcagcagg ggaatgtctt ttcttgctct gtcatgcatg aggctctgca taaccactac 1380

actcagaaat ccctgtctct gtctcccggg aaatgatagt aaaagctttg a 1431

<210> 11
<211> 1426
<212> DNA
<213> Artificial Sequence

<220>
<223> 2B1 H

<400> 11
gcggccgcca tgaattttgg actgaggctg attttcctgg tgctgaccct gaaaggcgtc 60

cagtgtcaag tccagctcgt ccagagcggg gcggaagtca agaagccggg ggcgagcgtg 120

aaagtctcgt gcaaggcgag cggatatacg ttcacgaact actggattca ctgggtccgg 180

caagcgccgg ggcaggggct ggagtggatc gggaacatcg acccgtcgga cacgcggacg 240

cactacaacc agaagttcaa ggaccgggcg acgctgaccg tcgacaagag cacgagcacg 300

gcgtacatgg agctgtcgag cctgcggagc gaggacacgg ccgtctacta ctgcgcgcgg 360

cagacgctgt actacgaggc gctggactac tgggggcagg ggacgctcgt cacggtctcg 420

agcgctagca caaagggccc tagtgtgttt cctctggctc cctcttccaa atccacttct 480

ggtggcactg ctgctctggg atgcctggtg aaggattact tccctgaacc tgtgactgtc 540

tcatggaact ctggtgctct gacttctggt gtccacactt tccctgctgt gctgcagtct 600

agtggactgt actctctgtc atctgtggtc actgtgccct cttcatctct gggaacccag 660

acctacattt gtaatgtgaa ccacaaacca tccaacacta aagtggacaa aagagtggaa 720

cccaaatcct gtgacaaaac ccacacctgc ccaccttgtc cggcgcctga agcggaagga 780

ggaccttctg tgtttctgtt cccccccaaa ccaaaggata ccctgatgat ctcgcgaacc 840

cctgaggtga catgtgtggt ggtggatgtg tctcatgagg accccgaagt caaatttaat 900

tggtatgtcg acggcgtcga ggtgcataat gccaaaacca agcctagaga ggaacagtac 960

aattcaacct acagagtcgt cagtgtgctg actgtgctgc atcaggattg gctgaatggc 1020

aaggaataca agtgtaaagt ctcaaacaag gccctgcctg ctccaattga gaaaacaatc 1080

tcaaaggcca agggacagcc tagggaaccc caggtctaca ccctgccacc ttcaagagag 1140

gaaatgacca aaaaccaggt gtccctgaca tgcctggtca aaggcttcta cccttctgac 1200

attgctgtgg agtgggagtc aaatggacag cctgagaaca actacaaaac aaccccccct 1260

gtgctggatt ctgatggctc tttctttctg tactccaaac tgactgtgga caagtctaga 1320

tggcagcagg ggaatgtctt ttcttgctct gtcatgcatg aggctctgca taaccactac 1380

actcagaaat ccctgtctct gtctcccggg aaatgataag ctttga 1426

<210> 12
<211> 1503
<212> DNA
<213> Artificial Sequence

<220>
<223> 2B2 H

<400> 12
gcggccgcca tgaattttgg actgaggctg attttcctgg tgctgaccct gaaaggcgtc 60

cagtgtcaag tccagctcgt ccagagcggg gcggaagtca agaagccggg ggcgagcgtg 120

aaagtctcgt gcaaggcgag cggatatacg ttcacgaact actggattca ctgggtccgg 180

caagcgccgg ggcaggggct ggagtggatc gggaacatcg acccgtcgga cacgcggacg 240

cactacaacc agaagttcaa ggaccgggcg acgctgaccg tcgacaagag cacgagcacg 300

gcgtacatgg agctgtcgag cctgcggagc gaggacacgg ccgtctacta ctgcgcgcgg 360

cagacgctgt actacgaggc gctggactac tgggggcagg ggacgctcgt cacggtctcg 420

agcgctagca caaagggccc tagtgtgttt cctctggctc cctcttccaa atccacttct 480

ggtggcactg ctgctctggg atgcctggtg aaggattact tccctgaacc tgtgactgtc 540

tcatggaact ctggtgctct gacttctggt gtccacactt tccctgctgt gctgcagtct 600

agtggactgt actctctgtc atctgtggtc actgtgccct cttcatctct gggaacccag 660

acctacattt gtaatgtgaa ccacaaacca tccaacacta aagtggacaa aagagtggaa 720

cccaaatcct gtgacaaaac ccacacctgc ccaccttgtc cggcgcctga agcggaagga 780

ggaccttctg tgtttctgtt cccccccaaa ccaaaggata ccctgatgat ctcgcgaacc 840

cctgaggtga catgtgtggt ggtggatgtg tctcatgagg accccgaagt caaatttaat 900

tggtatgtcg acggcgtcga ggtgcataat gccaaaacca agcctagaga ggaacagtac 960

aattcaacct acagagtcgt cagtgtgctg actgtgctgc atcaggattg gctgaatggc 1020

aaggaataca agtgtaaagt ctcaaacaag gccctgcctg cttcaattga gaaaacaatc 1080

tcaaaggcca agggacagcc tagggaaccc caggtctaca ccctgccacc ttcaagagag 1140

gaaatgacca aaaaccaggt gtccctgaca tgcctggtca aaggcttcta cccttctgac 1200

attgctgtgg agtgggagtc aaatggacag cctgagaaca actacaaaac aaccccccct 1260

gtgctggatt ctgatggctc tttctttctg tactccaaac tgactgtgga caagtctaga 1320

tggcagcagg ggaatgtctt ttcttgctct gtcatgcatg aggctctgca taaccactac 1380

actcagaaat ccctgtctct gtctcccggg aaatgataag ctttgatgtc atgcatgagg 1440

ctctgcataa ccactacact cagaaatccc tgtctctgtc tcccgggaaa tgataagctt 1500

tga 1503

<210> 13
<211> 19
<212> DNA
<213> Artificial Sequence

<220>
<223> Inhibitory Nucleic Acid

<400> 13
atgaaactcc ttgtgctag 19

<210> 14
<211> 14
<212> DNA
<213> Artificial Sequence

<220>
<223> Inhibitory Nucleic Acid

<400> 14
acagcggcat cagc 14

<210> 15
<211> 17
<212> DNA
<213> Artificial Sequence

<220>
<223> Inhibitory Nucleic Acid

<400> 15
ttccgcaaaa tgatcaa 17

<210> 16
<211> 16

<212>  DNA
<213>  Artificial Sequence

<220>
<223>  Inhibitory Nucleic Acid

<400>  16
cccggggagt gacccc                                                       16


<210>  17
<211>  20
<212>  DNA
<213>  Artificial Sequence

<220>
<223>  Inhibitory Nucleic Acid

<400>  17
tacggctgct actgtggctt                                                   20


<210>  18
<211>  23
<212>  DNA
<213>  Artificial Sequence

<220>
<223>  Inhibitory Nucleic Acid

<400>  18
gacacatgac aactgctacg acc                                               23


<210>  19
<211>  22
<212>  DNA
<213>  Artificial Sequence

<220>
<223>  Inhibitory Nucleic Acid

<400>  19
acccacacct attcatactc gt                                                22


<210>  20
<211>  16
<212>  DNA
<213>  Artificial Sequence

<220>
<223>  Inhibitory Nucleic Acid

<400>  20
atcacctgta gcagca                                                       16


<210>  21
<211>  19
<212>  DNA
<213>  Artificial Sequence

<220>
<223>  Inhibitory Nucleic Acid

<400>  21
agctccatat aacaaggca                                                    19


<210>  22
<211>  18
<212>  DNA
<213>  Artificial Sequence

<220>
<223>  Inhibitory Nucleic Acid

<400>  22
caagaagtat tgtcagag                                                     18


<210>  23
<211>  6
<212>  PRT
<213>  Artificial Sequence

<220>
<223>  Inhibitory Peptide

<400>  23

Asn Asn Tyr Gly Cys Tyr
1               5


<210>  24
<211>  6
<212>  PRT
<213>  Artificial Sequence

<220>
<223>  Inhibitory Peptide

<400>  24

Cys Tyr Cys Gly Leu Gly
1               5


<210>  25
<211>  14
<212>  PRT
<213>  Artificial Sequence

<220>
<223>  Inhibitory Peptide

<400>  25

Tyr Asn Asn Tyr Gly Cys Tyr Cys Gly Leu Gly Gly Ser Gly
1               5                   10


<210>  26
<211>  20

<212>  PRT
<213>  Artificial Sequence

<220>
<223>  Inhibitory Peptide

<400>  26

Phe Leu Glu Tyr Asn Asn Tyr Gly Cys Tyr Cys Gly Leu Gly Gly Ser
1               5                   10                  15

Gly Thr Pro Val
            20

<210>  27
<211>  5
<212>  PRT
<213>  Artificial Sequence

<220>
<223>  Inhibitory Peptide

<400>  27

Gln Thr His Asp Asn
1               5

<210>  28
<211>  7
<212>  PRT
<213>  Artificial Sequence

<220>
<223>  Inhibitory Peptide

<400>  28

Cys Gln Thr His Asp Asn Cys
1               5

<210>  29
<211>  9
<212>  PRT
<213>  Artificial Sequence

<220>
<223>  Inhibitory Peptide

<400>  29

Glu Cys Glu Ala Phe Ile Cys Asn Cys
1               5

<210>  30
<211>  6
<212>  PRT
<213>  Artificial Sequence

<220>
<223>  Inhibitory Peptide

<400>  30

Asp Arg Asn Ala Ala Ile
1               5


<210>  31
<211>  20
<212>  PRT
<213>  Artificial Sequence

<220>
<223>  Inhibitory Peptide

<400>  31

Asp Arg Asn Ala Ala Ile Cys Phe Ser Lys Ala Pro Tyr Asn Lys Ala
1               5                   10                  15


His Lys Asn Leu
            20


<210>  32
<211>  5
<212>  PRT
<213>  Artificial Sequence

<220>
<223>  Inhibitory Peptide

<400>  32

Phe Leu Ser Tyr Lys
1               5


<210>  33
<211>  5
<212>  PRT
<213>  Artificial Sequence

<220>
<223>  Inhibitory Peptide

<400>  33

Phe Leu Ser Tyr Arg
1               5


<210>  34
<211>  5
<212>  PRT
<213>  Artificial Sequence

<220>
<223>  Inhibitory Peptide

<220>
<221>  MISC_FEATURE
<222>  (1)..(1)
<223>  X is 2-NaphtylAlanine

<220>
<221>  MISC_FEATURE
<222>  (4)..(4)
<223>  X is 2-NaphtylAlanine

<400>  34

Xaa Leu Ser Xaa Arg
1               5

## Description

### FIELD OF THE INVENTION

The present invention relates to compositions and methods for treating cancers in a subject in need thereof. The invention more particularly relates to methods of treating cancers by inhibiting PLA2GIB in a subject in need thereof.

### INTRODUCTION

The group IB pancreatic secreted phospholipase A2 (PLA2-GIB) is a low molecular weight (14 kD), highly stable (7 disulfide bonds), secreted protein, that catalyzes the hydrolysis of the sn-2 fatty acyl bond of phospholipids to release free fatty acids and lysophospholipids (Lambeau & Gelb 2008).

PLA2-GIB was identified by the inventor as involved in CD4 T inactivation in HIV infected patients (WO2015/097140).

Continuing their research, the inventors have found the presence of PLA2-GIB cofactors in patients with cancers. The inventors have shown that such cofactors, together with PLA2-GIB, induce inactivation of immune cells in cancer patients, thereby allowing the tumor to escape an immune response. The inventors have further shown that inhibiting PLA2-GIB can restore an effective immune response and thus efficiently contribute to cancer treatment.

### SUMMARY OF THE INVENTION

An object of the invention relates to a method for treating cancer in a subject, comprising exposing the subject to a compound that inhibits PLA2-GIB.

A further object of the invention relates to a compound that inhibits PLA2-GIB for use for treating cancer in a subject.

A further object of the invention relates to the use of a compound that inhibits PLA2-GIB for the manufacture of a medicament for treating cancer in a subject.

The compound that inhibits PLA2-GIB may be used alone or in combination with one or more other treatment(s), such as a further anticancer agent, surgery, vaccine, radiotherapy, ultrasound therapy, or an inhibitor of a cofactor of PLA2-GIB.

The invention may be used for treating any tumor or cancer, such as particularly pancreatic cancer, melanoma, lung, oesophageal or pharyngeal cancer, retinoblastoma, liver, breast, ovary, renal, gastric, duodenum, uterine, cervical, thyroid, bladder, prostate, bone, brain or colorectal cancer. The invention may be used in any mammal, particularly in human subjects.

### BRIEF DESCRIPTION OF THE DRAWINGS

Fig. 1: Determination of PLA2-GIB concentrations (pro-, active and total) in plasma from PDAC cohort in comparison to control cohort.
Figure 2: In vitro effect of PDAC plasma on IL-7 response of CD4 T cells from healthy donor (A). Effect of a PLA2-GIB inhibitor (B).
Figure 3: In vitro effect of PDAC plasma on IL-2 response of CD4 T cells from healthy donor (A). Effect of a PLA2-GIB inhibitor (B)

### DETAILED DESCRIPTION OF THE INVENTION

The present invention relates to compositions and methods for treating cancers and neoplasia in a subject in need thereof.

### Definitions

As used herein, "treatment" or "treat" refers to clinical intervention in an attempt to alter the natural course of the individual being treated, and can be performed either for preventive or curative purpose. Desirable effects of cancer treatment include, but are not limited to, preventing occurrence or recurrence of cancer, alleviation of symptoms, preventing and treating metastasis, decreasing or slowing (the rate of) cancer progression, ameliorating or palliating the cancer state, causing or allowing cancer remission, or causing or inducing cancer cell destruction, or providing synergistic effect to other anti-cancer therapies. Treatment also encompasses any delay in cancer development.

A "subject" refers to a mammal. Examples of mammals include humans and non-human animals such as, without limitation, domesticated animals (e.g., cows, sheep, cats, dogs, and horses), non-human primates (such as monkeys), rabbits, and rodents (e.g., mice and rats). The invention is particularly suited for treating humans.

The term "isolated", as used herein, refers to molecules (e.g., nucleic or amino acid) that are removed from a component of their natural environment, isolated or separated, and are at least 60% free, preferably 75% free, and most preferably 90% free from other components with which they are naturally associated. An "isolated" polypeptide (or protein) is for instance a polypeptide separated from a component of its natural environment and, preferably purified to greater than 90% or 95% purity as determined by, for example, electrophoretic (e.g., SDS-PAGE, isoelectric focusing (IEF), capillary electrophoresis) or chromatographic (e.g., ion exchange or reverse phase HPLC) migration. An "isolated" nucleic acid refers to a nucleic acid molecule separated from a component of its natural environment and/or assembled in a different construct (e.g., a vector, expression cassette, recombinant host, etc.).

The term "sequence identity" as applied to nucleic acid or protein sequences, refers to the quantification (usually percentage) of nucleotide or amino acid residue matches between at least two sequences aligned using a standardized algorithm such as Smith-Waterman alignment (Smith and Waterman (1981) J Mol Biol 147:195-197), CLUSTALW (Thompson et al. (1994) Nucleic Acids Res 22:4673-4680), or BLAST2 (Altschul et al. (1997) Nucleic Acids Res 25:3389-3402). BLAST2 may be used in a standardized and reproducible way to insert gaps in one of the sequences in order to optimize alignment and to achieve a more meaningful comparison between them.

### PLA2-GIB

As used herein, "PLA2-GIB" (or "PLA2GIB" or "GIBsPLA2") designates group IB pancreatic phospholipase A2. PLA2-GIB has been identified and cloned from various mammalian species. The human PLA2-GIB protein is disclosed, for instance, in Lambeau and Gelb (2008). The sequence thereof is available on Genbank No. NP_000919. The amino acid sequence of an exemplary human PLA2-GIB is shown below as SEQ ID NO: 1.

MKLLVLAVLL TVAAADSGIS PRAVWQFRKM IKCVIPGSDP FLEYNNYGCY
CGLGGSGTPV DELDKCCQTH DNCYDQAKKL DSCKFLLDNP YTHTYSYSCS
GSAITCSSKN KECEAFICNC DRNAAICFSK APYNKAHKNL DTKKYCQS (SEQ 1)

Amino acids 1 to 15 of SEQ ID NO: 1 (underlined) are a signal sequence, and amino acids 16 to 22 of SEQ ID NO: 1 (in bold) are a propeptide sequence. A mature form of PLA2-GIB is produced typically by proteolytic cleavage and is thus devoid of signal sequence and propeptide sequence. A typical example of a mature form of PLA2-GIB thus comprises (or consists essentially of) amino acid residues 23-148 of SEQ ID NO: 1, as represented in SEQ ID NO: 2 below.

AVWQFRKMIK CVIPGSDPFL EYNNYGCYCG LGGSGTPVDE LDKCCQTHDN
CYDQAKKLDS CKFLLDNPYT HTYSYSCSGS AITCSSKNKE CEAFICNCDR
NAAICFSKAP YNKAHKNLDT KKYCQS (SEQ ID NO: 2)

Within the context of the invention, the term "PLA2-GIB" designates preferably a human PLA2-GIB, in any form thereof (pro- or mature), as well as variants thereof such as any natural variants resulting from polymorphism or splicing.

In a particular embodiment, the term "PLA2-GIB" designates a human PLA2-GIB comprising SEQ ID NO: 1 or 2 and natural variants resulting from polymorphism or splicing.

Naturally-occurring variants of a reference protein include any protein with one or more amino acid substitutions, additions and/or deletions of one or several (typically 1, 2 or 3) amino acid residues, as compared to said reference protein. Preferably, the variant includes not more than 10 distinct amino acid substitution(s), addition(s), and/or deletion(s) of one or several (typically 1, 2 or 3) amino acid residues as compared to the reference protein. Typical naturally-occurring variants retain a biological activity of the reference protein. In this regard, in some embodiments, PLA2-GIB has at least one activity selected from induction of formation of membrane microdomains (MMD) in CD4 T cells from healthy subjects, or rendering CD4 T cells of healthy subjects refractory to interleukin signaling, such as refractory to IL-2 signaling or refractory to IL-7 signaling.

In a particular embodiment, the term PLA2-GIB designates a human protein, particularly a protein comprising or consisting of SEQ ID NO: 1 or 2, or a naturally-occurring variant thereof.

### Treatment of cancers

The invention relates to methods for treating cancer in a subject comprising administering to the subject a compound that inhibits PLA2-GIB. The inventors have shown that PLA2-GIB cofactors exist in patients having cancer which, together with PLA2-GIB, induce inactivation of immune cells. The inventors have further shown that inhibiting PLA2-GIB can restore an effective immune response in the plasma of said patients and thus efficiently contribute to cancer treatment.

In a particular embodiment, the invention relates to methods for treating cancer or neoplasia in a subject in need thereof, comprising administering to the subject a compound that inhibits PLA2-GIB.

The invention also relates to a compound that inhibits PLA2-GIB for use for treating cancer or neoplasia in a subject in need thereof.

In a particular embodiment, the method of the invention is for preventing cancer or reducing the rate of cancer occurrence in a subject in need thereof, such as a subject at risk of neoplasia or cancer. In this regard, the invention can be used for treating risk factors for cancers, thereby avoiding or reducing the risk/rate of occurrence of a cancer. Such risk factors include, without limitation, oro-, gastro-, and/or intestinal inflammation and infections, such as pancreatitis.

The invention also relates to a compound that inhibits PLA2-GIB for use for preventing cancer or reducing the rate of cancer occurrence in a subject in need thereof.

In another particular embodiment, the method of the invention is for reducing the rate of cancer progression in a subject having a cancer.

In another particular embodiment, the invention relates to a compound that inhibits PLA2-GIB for use for reducing the rate of cancer progression in a subject having a cancer.

In another particular embodiment, the method of the invention is for reducing or preventing or treating cancer metastasis in a subject having a cancer, or for killing cancer cells.

In another particular embodiment, the invention relates to a compound that inhibits PLA2-GIB for use for reducing or preventing or treating cancer metastasis in a subject having a cancer, or for killing cancer cells in a subject having a cancer.

The inventors have analyzed several cohorts of patients with solid tumor, such as pancreatic cancer. They have found that PLA2-GIB levels are not statistcally different in plasma from said patients as compared to plasma from healthy donors. However, they have surprisingly found that plasma from said patients can render immune cells sensitive to inactivation by physiological concentrations of PLA2-GIB. More specifically, by exposing T cells to plasma from said cancer patients and PLA2-GIB, said immune cells become inactive and unable to initiate an immune response. The plasma of said patients thus contain one or more cofactors which render immue cells sensitive to PLA2-GIB inactivation. The inventors have further shown that by inhibiting PLA2-GIB, said immune cells are restored and no such inactivation occurs.

The invention thus demonstrates the existence of PLA2-GIB cofactors in the plasma of human patients with cancer.

The invention further demonstrates that PLA2-GIB inhibition can be used to treat such cancers.

In this regard, the invention may be used for treating any cancer.

In a particular embodiment, the cancer is a solid cancer.

In a particular embodiment, the method is used for treating a subject having cancer and expressing a PLA2-GIB cofactor. In a preferred embodiment, the method is used for treating cancer in a subject, wherein a PLA2-GIB cofactor or a prokaryotic or eukaryotic cell or virus expressing a PLA2-GIB cofactor is present in said subject.

In another particular embodiment, the method is used for treating a subject having cancer, wherein PLA2-GIB or a PLA2-GIB cofactor is present in the cancer microenvironment or blood.

The invention is also particularly suitable for treating cancers or neoplasia in subjects having a PLA2GIB-related CD4 T cell deficiency.

The invention may be used to treat cancers at any stage of development. In this regard, most solid cancer develop through four stages:
- Stage I. This stage is usually a small cancer or tumor that has not grown deeply into nearby tissues. It also has not spread to the lymph nodes or other parts of the body. It is often called early-stage cancer.
- Stage II and Stage III. In general, these 2 stages indicate larger cancers or tumors that have grown more deeply into nearby tissue. They may have also spread to lymph nodes but not to other parts of the body.
- Stage IV. This stage means that the cancer has spread to other organs or parts of the body. It may also be called advanced or metastatic cancer.

Some cancers also have a stage 0. Stage 0 cancers are still located in the place they started and have not spread to nearby tissues. This stage of cancer is often highly curable, usually by removing the entire tumor with surgery.

The invention may be used for treating tumors or cancers at stage 0, I, II, III or IV.

The invention may be used to prevent or reduce or treat metastasis of a cancer at stage 0, I, II or III.

The invention may be used to reduce the rate of progression of a cancer at stage 0, I, II, III or IV.

The invention may in particular be used for treating solid cancers selected from pancreatic cancer, melanoma, lung, oesophageal or pharyngeal cancer, retinoblastoma, liver, breast, ovary, renal, gastric, duodenum, uterine, cervical, thyroid, bladder, prostate, bone, brain or colorectal cancer.

In a specific embodiment, the method of the invention is for treating pancreatic cancer. Pancreatic cancer is classified according to which part of the pancreas is affected: the part that makes digestive substances cause exocrine cancers, the part that makes insulin and other hormones cause endocrine cancers. Although there are several different types of pancreatic cancer, 95% of cases are due to an exocrine cancer, the pancreatic ductal adenocarcinoma (PDAC).

PDAC is ranked the fourth among the major cause of death due to cancer. PDAC is projected by researchers to become the second-most leading cause of cancer-related death in the US by 2030. Incidence has more than doubled in 30 years and currently increases by 5% annually. The relative survival rate for 5 years is around 5% and surgical operation is the most efficient option for the treatment of PDAC. The limited availability of diagnostic approaches, and surgery as the solely existing curative option with the survival possibility of only 10% of diagnostic patients, increases the dreadfulness of this disease. The poor prognosis of the disease can be explained by the absence of effective biomarkers for screening and early detection, together with the aggressive behavior and resistance to the currently available chemotherapy.

The invention shows PLA2-GIB inhibition can be used to treat pancreatic cancer. The invention represents a new strategy to prevent pancreatic cancer progression and metastasis. The invention may be used with any type / stage of pancreatic cancer, such as pancreatic adenocarcinoma, neuroendocrine tumor, intraductal papillary-mucinous neoplasama, mucinous cystic neoplasm, and serious cystic neoplasm. The invention is particulary suited for treating pancreatic adenocarcimona, at any stage.

The invention is also particularly suitede for treating colorectal cancer, lung cancer, as well as fast-growing cancers. Colorectal cancer is one of the most common cancer of all genders. At all stages, the probability of survival at 5 years is about 55%. (Bossard N, 2007). Indeed, in France, Japan, US, Germany, Italy, Spain and the United Kingdom, more than 180 000 new cases of rectal cancer were diagnosed in 2010. Colorectal cancer is classified into four stages: stage I, which is the least advanced and is primarily managed by surgery, stages II and III, for which patients undergo combined radiochemotherapy (RCT), and stage IV, which is a very advanced and metastasized stage. When a patient is diagnosed with locally advanced (stage II or III) colorectal cancer, the patient is typically treated with RCT prior to surgical resection. The invention is suited for treating stage I, II, III and IV colorectal cancer. The invention is particularly suited for teating colorectal cancer at stage II, III or IV.

The invention is also suitable for treating cancer that induce gastrointestinal and metabolic pathologies.

For use in the present invention, the PLA2-GIB inhibitor may be administered by any suitable route. Preferably, administration is by injection, such as systemic or parenteral injection or perfusion, e.g., intramuscular, intravenous, intraarterial, subcutaneous, intratumoral, etc. Administration is typically repeated, or continuous. In a particular embodiment, the level of PLA2-GIB or PLA2-GIB cofactor in the tumor or in body fluids is measured during the course of treatment to guide therapeutic regimen.

The PLA2-GIB inhibitor may be used alone, or in combination with further cancer treatment(s).

In a particular embodiment, the invention relates to a method for treating cancer in a subject comprising administering to the suject having a cancer a compound that inhibits PLA2-GIB in combination with chemotherapy or hormonotherapy.

In another particular embodiment, the invention relates to a method for treating cancer in a subject comprising administering to the suject having a cancer a compound that inhibits PLA2-GIB in combination with radiotherapy, ultrasound therapy or nanoparticle therapy.

In another particular embodiment, the invention relates to a method for treating cancer in a subject comprising administering to the suject having a cancer a compound that inhibits PLA2-GIB in combination with a check-point inhibitor, immunotherapy or an anti-cancer vaccine.

In another particular embodiment, the invention relates to a method for treating cancer in a subject comprising administering to the suject having a cancer a compound that inhibits PLA2-GIB in combination with an inhibitor of a cofactor of PLA2-GIB. The inhibitor of the PLA2-GIB cofactor may be an antagonist of said cofactor, or a cytostatic or cytotoxic agent or a vaccine against said PLA2-GIB cofactor or against a prokaryotic or eukaryotic cell or virus expressing said PLA2-GIB cofactor. In this regard, where the cofactor is a bacterium, the inhibitor may be an antibiotic agaist said bacterium.

In a "combination" therapy, the active agents may be used simultaneoysly or sequentially, together or in alternance. Each active agent may be used according to a specific schedule. In other instances, all active agents may be formulated and/or administered together, such as in a perfusion.

In a further embodiment, the compound is administered prior to, during or after surgery (tumor resection or removal).

The compounds for use in the present invention may be formulated in a pharmaceutical composition comprising one or more pharmaceutically acceptable carriers.

A "pharmaceutical composition" refers to a formulation of a compound of the invention (active ingredient) and a medium generally accepted in the art for the delivery of biologically active compounds to the subject in need thereof. Such a carrier includes all pharmaceutically acceptable carriers, diluents, medium or supports therefore. Conventional pharmaceutical practice may be employed to provide suitable formulations or compositions to subjects, for example in unit dosage form.

The compounds or compositions according to the invention may be formulated in the form of ointment, gel, paste, liquid solutions, suspensions, tablets, gelatin capsules, capsules, suppository, powders, nasal drops, or aerosol, preferably in the form of an injectible solution or suspension. For injections, the compounds are generally packaged in the form of liquid suspensions, which may be injected via syringes or perfusions, for example. In this respect, the compounds are generally dissolved in saline, physiological, isotonic or buffered solutions, compatible with pharmaceutical use and known to the person skilled in the art. Thus, the compositions may contain one or more agents or excipients selected from dispersants, solubilizers, stabilizers, preservatives, etc. Agents or excipients that can be used in liquid and/or injectable formulations are notably methylcellulose, hydroxymethylcellulose, carboxymethylcellulose, polysorbate 80, mannitol, gelatin, lactose, vegetable oils, acacia, etc. The carrier can also be selected for example from methyl-betacyclodextrin, a polymer of acrylic acid (such as carbopol), a mixture of polyethylene glycol and polypropylene glycol, monoetrhanol amine and hydroxymethyl cellulose.

The compositions generally comprise from about 1 µg to 1000 mg of a PLA2-GIB inhibitor, such as from 0.001-0.01, 0.01-0.1, 0.05-100, 0.05-10, 0.05-5, 0.05-1, 0.1-100, 0.1-1.0, 0.1-5, 1.0-10, 5-10, 10-20, 20-50, and 50-100 mg, for example between 0.05 and 100 mg, preferably between 0.05 and 5 mg, for example 0.05, 0.1, 0.2, 0.3, 0.4, 0.5, 1, 2, 3, 4 or 5 mg. The dosage may be adjusted by the skilled person depending on the modulator and the disease.

The compositions may be formulated in any suitable form or container (syringe, apoule, flask, bottle, pouch, etc).

In a preferred embodemnt, the treatment comprises one or several injection(s) of a liquid formulation containing a PLA2-GIB inhibitor, optionally in combination with one or more anticancer agent(s) or treatment(s). Injections are preferably systemic, such as intravenous, intraarterial, intramuscular, intracancerous, intradermic, etc.

The dosage and frequency of administration can be adjusted by the practitioner.

### Inhibitors of PLA2-GIB

PLA2-GIB inhibitors suitable for use in the invention may be any compound that inhibits or neutralizes the expression or activity of PLA2-GIB, such as expression inhibitors, antagonists, or sequestrators. Preferred types of inhibitors include PLA2-GIB ligands (covalent or non-covalent), anti-PLA2-GIB antibodies (and fragments and derivatives thereof), nucleic acids encoding anti-PLA2-GIB antibodies (or fragments and derivatives thereof), inhibitory nucleic acids, peptides, or small drugs, soluble receptors, or combination(s) thereof. Alternatively, or in addition, the PLA2-GIB inhibitor can be a PLA2-GIB antigen which, uon administration to the subject, induces the production of anti-PLA2GIB antibodies.

Inhibiting PLA2-GIB designates typically reducing by at least 10%, 20%, 30%, 40%, 50%, 60%, 70%, 80% or more PLA2-GIB level or activity, as well as completely blocking or suppressing PLA2-GIB level or activity. Depending on the situation, inhibition may be transient, sustained or permanent.

### Antibodies against PLA2-GIB

Specific examples of PLA2-GIB inhibitors are anti-PLA2-GIB antibodies, e.g., antibodies that bind to PLA2-GIB and/or have been generated by immunization of a mammal with a PLA2-GIB antigen.

Antibodies can be synthetic, monoclonal, or polyclonal and can be made by techniques well known in the art. Such antibodies specifically bind via the antigen-binding sites of the antibody (as opposed to non-specific binding). PLA2-GIB polypeptides, fragments, variants, fusion proteins, etc., can be employed as immunogens in producing antibodies immunoreactive therewith. More specifically, the polypeptides, fragments, variants, fusion proteins, etc. contain antigenic determinants or epitopes that elicit the formation of antibodies.

The term "antibodies" is meant to include polyclonal antibodies, monoclonal antibodies, fragments thereof, such as F(ab')2 and Fab fragments, single-chain variable fragments (scFvs), single-domain antibody fragments (VHHs or Nanobodies), bivalent antibody fragments (diabodies), as well as any recombinantly and synthetically produced binding partners, human antibodies or humanized antibodies.

Antibodies are defined to be specifically binding preferably if they bind to PLA2-GIB with a Ka of greater than or equal to about 10⁷ M-1. Affinities of antibodies can be readily determined using conventional techniques, for example those described by Scatchard et al., Ann. N.Y. Acad. Sci., 51:660 (1949).

Polyclonal antibodies can be readily generated from a variety of sources, for example, horses, cows, donkeys, goats, sheep, dogs, chickens, rabbits, mice, or rats, using procedures that are well known in the art. In general, purified PLA2-GIB or a peptide based on the amino acid sequence of PLA2-GIB that is appropriately conjugated is administered to the host animal typically through parenteral injection. The immunogenicity of PLA2-GIB can be enhanced through the use of an adjuvant, for example, Freund's complete or incomplete adjuvant. Following booster immunizations, small samples of serum are collected and tested for reactivity to PLA2-GIB polypeptide. Examples of various assays useful for such determination include those described in Antibodies: A Laboratory Manual, Harlow and Lane (eds.), Cold Spring Harbor Laboratory Press, 1988; as well as procedures, such as countercurrent immuno-electrophoresis (CIEP), radioimmunoassay, radioimmunoprecipitation, enzyme-linked immunosorbent assays (ELISA), dot blot assays, and sandwich assays. See U.S. Pat. Nos. 4,376,110 and 4,486,530.

Monoclonal antibodies can be readily prepared using well known procedures. See, for example, the procedures described in U.S. Pat. Nos. RE 32,011, 4,902,614, 4,543,439, and 4,411,993; Monoclonal Antibodies, Hybridomas: A New Dimension in Biological Analyses, Plenum Press, Kennett, McKeam, and Bechtol (eds.), 1980.

For example, the host animals, such as mice, can be injected intraperitoneally at least once and preferably at least twice at about 3 week intervals with isolated and purified wild-type or mutant PLA2-GIB protein or conjugated PLA2-GIB peptide, optionally in the presence of adjuvant. Mouse sera are then assayed by conventional dot blot technique or antibody capture (ABC) to determine which animal is best to fuse. Approximately two to three weeks later, the mice are given an intravenous boost of protein or peptide. Mice are later sacrificed and spleen cells fused with commercially available myeloma cells, such as Ag8.653 (ATCC), following established protocols. Briefly, the myeloma cells are washed several times in media and fused to mouse spleen cells at a ratio of about three spleen cells to one myeloma cell. The fusing agent can be any suitable agent used in the art, for example, polyethylene glycol (PEG). Fusion is plated out into plates containing media that allows for the selective growth of the fused cells. The fused cells can then be allowed to grow for approximately eight days. Supernatants from resultant hybridomas are collected and added to a plate that is first coated with goat anti-mouse Ig. Following washes, a label, such as a labeled PLA2-GIB polypeptide, is added to each well followed by incubation. Positive wells can be subsequently detected. Positive clones can be grown in bulk culture and supernatants are subsequently purified over a Protein A column (Pharmacia).

The monoclonal antibodies of the disclosure can be produced using alternative techniques, such as those described by Alting-Mees et al., "Monoclonal Antibody Expression Libraries: A Rapid Alternative to Hybridomas", Strategies in Molecular Biology 3:1-9 (1990), which is incorporated herein by reference. Similarly, binding partners can be constructed using recombinant DNA techniques to incorporate the variable regions of a gene that encodes a specific binding antibody. Such a technique is described in Larrick et al., Biotechnology, 7:394 (1989).

Antigen-binding fragments of such antibodies, which can be produced by conventional techniques, are also encompassed by the present invention. Examples of such fragments include, but are not limited to, Fab and F(ab')2 fragments. Antibody fragments and derivatives produced by genetic engineering techniques are also provided.

The monoclonal antibodies include chimeric antibodies, e.g., humanized versions of murine monoclonal antibodies. Such humanized antibodies can be prepared by known techniques, and offer the advantage of reduced immunogenicity when the antibodies are administered to humans. In one embodiment, a humanized monoclonal antibody comprises the variable region of a murine antibody (or just the antigen binding site thereof) and a constant region derived from a human antibody. Alternatively, a humanized antibody fragment can comprise the antigen binding site of a murine monoclonal antibody and a variable region fragment (lacking the antigen-binding site) derived from a human antibody. Procedures for the production of chimeric and further engineered monoclonal antibodies include those described in Riechmann et al. (Nature 332:323, 1988), Liu et al. (PNAS 84:3439, 1987), Larrick et al. (Bio/Technology 7:934, 1989), and Winter and Harris (TIPS 14:139, May, 1993). Procedures to generate antibodies transgenically can be found in GB 2,272,440, U.S. Pat. Nos. 5,569,825 and 5,545,806.

Antibodies produced by genetic engineering methods, such as chimeric and humanized monoclonal antibodies, comprising both human and non-human portions, which can be made using standard recombinant DNA techniques, can be used. Such chimeric and humanized monoclonal antibodies can be produced by genetic engineering using standard DNA techniques known in the art, for example using methods described in Robinson et al. International Publication No. WO 87/02671; Akira, et al. European Patent Application 0184187; Taniguchi, M., European Patent Application 0171496; Morrison et al. European Patent Application 0173494; Neuberger et al. PCT International Publication No. WO 86/01533; Cabilly et al. U.S. Pat. No. 4,816,567; Cabilly et al. European Patent Application 0125023; Better et al., Science 240:1041 1043, 1988; Liu et al., PNAS 84:3439 3443, 1987; Liu et al., J. Immunol. 139:3521 3526, 1987; Sun et al. PNAS 84:214 218, 1987; Nishimura et al., Canc. Res. 47:999 1005, 1987; Wood et al., Nature 314:446 449, 1985; and Shaw et al., J. Natl. Cancer Inst. 80:1553 1559, 1988); Morrison, S. L., Science 229:1202 1207, 1985; Oi et al., BioTechniques 4:214, 1986; Winter U.S. Pat. No. 5,225,539; Jones et al., Nature 321:552 525, 1986; Verhoeyan et al., Science 239:1534,1988; and Beidler et al., J. Immunol. 141:4053 4060, 1988.

In connection with synthetic and semi-synthetic antibodies, such terms are intended to cover but are not limited to antibody fragments, isotype switched antibodies, humanized antibodies (e.g., mouse-human, human-mouse), hybrids, antibodies having plural specificities, and fully synthetic antibody-like molecules.

Human monoclonal antibodies having human constant and variable regions can be generated by immunizing transgenic animals which contain human immunoglobulin genes. See Jakobovits et al. Ann NY Acad Sci 764:525-535 (1995). Human monoclonal antibodies against PLA2-GIB polypeptides can also be prepared by constructing a combinatorial immunoglobulin library, such as a Fab phage display library or a scFv phage display library, using immunoglobulin light chain and heavy chain cDNAs prepared from mRNA derived from lymphocytes of a subject. See, e.g., McCafferty et al. PCT publication WO 92/01047; Marks et al. (1991) J. Mol. Biol. 222:581 597; and Griffths et al. (1993) EMBO J 12:725 734. In addition, a combinatorial library of antibody variable regions can be generated by mutating a known human antibody. For example, a variable region of a human antibody known to bind PLA2-GIB, can be mutated by, for example, using randomly altered mutagenized oligonucleotides, to generate a library of mutated variable regions which can then be screened to bind to PLA2-GIB. Methods of inducing random mutagenesis within the CDR regions of immunoglobin heavy and/or light chains, methods of crossing randomized heavy and light chains to form pairings and screening methods can be found in, for example, Barbas et al. PCT publication WO 96/07754; Barbas et al. (1992) Proc. Nat'l Acad. Sci. USA 89:4457 4461.

An immunoglobulin library can be expressed by a population of display packages, preferably derived from filamentous phage, to form an antibody display library. Examples of methods and reagents particularly amenable for use in generating antibody display library can be found in, for example, Ladner et al. U.S. Pat. No. 5,223,409; Kang et al. PCT publication WO 92/18619; Dower et al. PCT publication WO 91/17271; Winter et al. PCT publication WO 92/20791; Markland et al. PCT publication WO 92/15679; Breitling et al. PCT publication WO 93/01288; McCafferty et al. PCT publication WO 92/01047; Garrard et al. PCT publication WO 92/09690; Ladner et al. PCT publication WO 90/02809; Fuchs et al. (1991) Bio/Technology 9:1370 1372; Hay et al. (1992) Hum Antibod Hybridomas 3:81 85; Huse et al. (1989) Science 246:1275 1281; Griffths et al. (1993) supra; Hawkins et al. (1992) J Mol Biol 226:889 896; Clackson et al. (1991) Nature 352:624 628; Gram et al. (1992) PNAS 89:3576 3580; Garrad et al. (1991) Bio/Technology 9:1373 1377; Hoogenboom et al. (1991) Nuc Acid Res 19:4133 4137; and Barbas et al. (1991) PNAS 88:7978 7982. Once displayed on the surface of a display package (e.g., filamentous phage), the antibody library is screened to identify and isolate packages that express an antibody that binds a PLA2-GIB polypeptide. In a preferred embodiment, the primary screening of the library involves panning with an immobilized PLA2-GIB polypeptide and display packages expressing antibodies that bind immobilized PLA2-GIB polypeptide are selected.

Preferred antibodies for use in the invention are directed to a PLA2-GIB epitope, and/or have been generated by immunization with a polypeptide comprising a PLA2-GIB epitope selected from: the mature PLA2-GIB protein, a fragment of PLA2-GIB comprising at least 8 consecutive amino acid residues of SEQ ID NO: 1 or 2 (or the corresponding residues of a natural variant of SEQ ID NO: 1 or 2), said fragment preferably comprising at least amino acid 70, amino acid 121, amino acid 50, amino acid 52, amino acid 54, amino acid 71, or a combination thereof (numbering by reference to SEQ ID NO: 2).

Particular anti-PLA2-GIB antibodies for use in the invention bind mature human PLA2-GIB, even more preferably an epitope comprised in a domain of PLA2-GIB comprising an amino acid residue selected from amino acid 70, amino acid 121, amino acid 50, amino acid 52, amino acid 54, amino acid 71, or a combination thereof (numbering by reference to SEQ ID NO: 2). Particular antibodies for use in the invention bind an epitope comprised between amino acid residues 50-71 of mature human PLA2-GIB (by reference to SEQ ID NO: 2) or the corresponding residues of a natural variant of SEQ ID NO: 2. Examples of anti-PLA2-GIB antibodies suitable for use in the invention have been disclosed in WO2015/097140.

Further particular anti-PLA2-GIB antibodies for use in the present invention bind an epitope comprising at least one amino acid residue selected from W3, R6, K7, K10, C77, Y75, G79 and S80 of human mature PLA2-GIB (numbering by reference to SEQ ID NO: 2), more preferably an epitope comprising at least 2 or at least 3 amino acid residues selected from W3, R6, K7, K10, C77, Y75, G79 and S80 of human mature PLA2-GIB, further more preferably an epitope comprising at least 4, at least 5, at least 6 or at least 7 amino acid residues selected from W3, R6, K7, K10, C77, Y75, G79 and S80 of human mature PLA2-GIB. Particular antibodies for use in the invention bind an epitope comprising an amino acid residue comprised between amino acids 1-10 or 75-80 of mature human PLA2-GIB (by reference to SEQ ID NO: 2) or the corresponding residues of a natural variant of SEQ ID NO: 2. Such antibodies exhibit potent neutralizing activity and represent valuable therapeutic agents for use in the invention. Examples of such anti-PLA2-GIB antibodies suitable for use in the invention have been disclosed in EP18305229, presently unpublished.

In a particular embodiment, the antibodies or derivatives for use in the invention is monoclonal antibody 14G9 or an anti-PLA2-GIB antibody that competitively inhibits binding of monoclonal antibody 14G9 to human PLA2-GIB, said monoclonal antibody 14G9 comprising a light chain variable region comprising SEQ ID NO: 3 and a heavy chain variable region comprising SEQ ID NO: 4. The antibody may be human or humanized.

In another particular embodiment, the antibodies or derivatives for use in the invention is monoclonal antibody #2B or an anti-PLA2-GIB antibody that competitively inhibits binding of monoclonal antibody #2B to human sPLA2-GIB, said monoclonal antibody #2B comprising a light chain comprising or consisting of SEQ ID NO: 5 and a heavy chain comprising or consisting of SEQ ID NO: 6.

In another particular embodiment, the antibodies or derivatives for use in the invention is monoclonal antibody #2B1 or an anti-PLA2-GIB antibody that competitively inhibits binding of monoclonal antibody #2B1 to human sPLA2-GIB, said monoclonal antibody #2B1 comprising a light chain comprising or consisting of SEQ ID NO: 5 and a heavy chain comprising or consisting of SEQ ID NO: 7.

In another particular embodiment, the antibodies or derivatives for use in the invention is monoclonal antibody #2B2 or an anti-PLA2-GIB antibody that competitively inhibits binding of monoclonal antibody #2B2 to human sPLA2-GIB, said monoclonal antibody #2B2 comprising a light chain comprising or consisting of SEQ ID NO: 5 and a heavy chain comprising or consisting of SEQ ID NO: 8.

The term "competitively inhibits" indicates that the antibody can reduce or inhibit or displace the binding of a reference antibody to sPLA2-GIB. Competition assays can be performed using standard techniques such as, for instance, competitive ELISA or other binding assays. Typically, a competitive binding assay involves a purified target antigen, generally bound either to a solid substrate or cells, an unlabeled test antibody and a labeled reference antibody. Competitive inhibition is measured by determining the amount of labeled antibody bound in the presence of the test antibody. Usually the test antibody is present in excess, such as about 5 to 500 times the amount of reference antibody. Typically, for ELISA, the test antibody is in 100X excess, and for enzymatic methods, the test antibody in in 10X excess. When a test antibody present in excess inhibits or displaces at least 70% of the binding of the reference antibody to the antigen, it is considered as competitively inhibiting said reference antibody. In a specific embodiment, when a test antibody present in 100X excess inhibits or displaces at least 70%, more preferably at least 80% of the binding of the reference antibody to the antigen in ELISA, it is considered as competitively inhibiting said reference antibody. Preferred competing antibodies bind epitopes that share common amino acid residues.

In a particular embodiment, the inhibitor is a monoclonal antibody comprising:
(i) a light chain variable region comprising a CDR-L1, a CDR-L2, a CDR-L3 and a FR-L, wherein the CDR-L1, CDR-L2 and/or CDR-L3 consists, or consists essentially, of the CDR-L1, CDR-L2 and CDR-L3, respectively, of the light chain variable region of SEQ ID NO: 3 or 5, and wherein a FR-L is of a human immunoglobulin sequence; and
(ii) a heavy chain variable region comprising a CDR-H1, a CDR-H2, a CDR-H3 and a FR-H, wherein the CDR-H1, CDR-H2 and/or CDR-H3 consists, or consists essentially, of the CDR-H1, CDR-H2 and CDR-H3, respectively, of the heavy chain variable region of SEQ ID NO: 4, 6, 7 or 8, and wherein a FR-H is of a human immunoglobulin sequence.

The "variable region" of an antibody refers to the amino-terminal domains of the heavy or light chain ("VH" or "VL"), which contain the antigen-binding sites. A light or heavy chain variable region (VL or VH) generally consists of a framework region ("FR") interrupted by three hypervariable regions referred to as "complementarity determining regions" or "CDRs". The extent of the framework region and CDRs have been precisely defined, for example as in Kabat (see "Sequences of Proteins of Immunological Interest," E. Kabat et al., U.S. Department of Health and Human Services, (1983)), and as in Chothia.

With reference to SEQ ID NO: 3, the three CDR regions correspond to the following amino acid residues:
- CDR-L1: amino acid residues QDVSTA (residues 27-31 of SEQ ID NO: 3),
- CDR-L2: amino acid residues WAS (residues 50-52 of SEQ ID NO: 3),
- CDR-L3: amino acid residues QQDYSTPPT (residues 89-97 of SEQ ID NO: 3).

With reference to SEQ ID NO: 4, the three CDR regions correspond to the following amino acid residues:
- CDR-H1: amino acid residues GYTFTNYW (residues 26-33 of SEQ ID NO: 4),
- CDR-H2: amino acid residues IDPSDTRT (residues 51-58 of SEQ ID NO: 4),
- CDR-H3: amino acid residues ARQTLYYEALDY (residues 97-108 of SEQ ID NO: 4).

In a particular embodiment, the invention relates to a monoclonal antibody selected from:
- a monoclonal antibody comprising a light chain comprising, consisting essentially of, or consisting of SEQ ID NO: 3 and a heavy chain comprising, consisting essentially of, or consisting of SEQ ID NO: 4 (14G9);
- a monoclonal antibody comprising a light chain comprising, consisting essentially of, or consisting of SEQ ID NO: 5 and a heavy chain comprising, consisting essentially of, or consisting of SEQ ID NO: 6 (#2B);
- a monoclonal antibody comprising a light chain comprising, consisting essentially of, or consisting of SEQ ID NO: 5 and a heavy chain comprising, consisting essentially of, or consisting of SEQ ID NO: 7 (#2B1);
- a monoclonal antibody comprising a light chain comprising, consisting essentially of, or consisting of SEQ ID NO: 5 and a heavy chain comprising, consisting essentially of, or consisting of SEQ ID NO: 8 (clone #2B2); and
- derivatives thereof.

The term "antibody derivative", as used herein, refers to an antibody which retains the antigenic specificity of a reference antibody but wherein one or more amino acid residues are (chemically, or biologically) modified to improve its properties.

Examples of such chemical modifications include, e.g. by alkylation, PEGylation, acylation, ester or amide formation, and the like. In particular, a derivative is an antibody as disclosed herein that is modified to contain one or more additional non-proteinaceous moieties such as water soluble polymers. Examples of water soluble polymers include, but are not limited to, PEG, copolymers of ethylene glycol/propylene glycol, carboxymethylcellulose, dextran and polyvinyl alcohol.

Derivatives may also be generated to increase or decrease the extent to which the antibody is glycosylated. Addition or deletion of glycosylation sites to an antibody may be conveniently accomplished by altering the amino acid sequence such that one or more glycosylation sites is created or removed. Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region (see e.g., Wright et al. TIBTECH, 1997, 15:26-32). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%). The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Examples of publications related to "defucosylated" or "fucose-deficient" antibody variants include, but are not limited to, Okazaki et al. J. Mol. Biol. 336: 1239-1249 (2004) and Yamane-Ohnuki N, Satoh M. mAbs. 2009;1:230-236. Examples of cell lines capable of producing defucosylated antibodies include Led 3 CHO cells deficient in protein fucosylation (Ripka et al. Arch. Biochem. Biophys. 249:533-545 (1986)), and knockout cell lines, such as alpha- 1 ,6-fucosyltransferase gene, FUT8, knockout CHO cells (see, e.g., Yamane-Ohnuki et al. Biotech. Bioeng. 87: 614 (2004); Kanda, Y. et al., Biotechnol. Bioeng., 94(4):680-688 (2006)).

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

The term derivative also includes immunoconjugates comprising an anti-sPLA2-GIB antibody as defined above conjugated to one or more heterologous molecule(s), including but not limited to a cytotoxic agent, a detectable moiety such as a fluorescent moiety, a diagnostic radioisotope or an imaging agent; or to a solid support, such as agarose beads or the like. Examples of cytotoxic agents include, but are not limited to chemotherapeutic agents or drugs, growth inhibitory agents, toxins (e.g., protein toxins, enzymatically active toxins of bacterial, fungal, plant, or animal origin, or fragments thereof), or radioactive isotopes. Conjugates of an antibody and cytotoxic agent may be made using a variety of bifunctional protein coupling agents well known by the skilled person. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52: 127-131 (1992)) may be used.

The antibodies for use in the invention are typically "isolated", e.g., have been separated from at least one component of their natural environment. In particular, the antibodies may be purified to greater e.g., at least 95%, at least 96%; at least 97%, at least 98% or at least 99% purity as determined by, for example, electrophoretic (e.g., SDS-PAGE, isoelectric focusing (IEF), capillary electrophoresis) or chromatographic (e.g., ion exchange or reverse phase HPLC) techniques. For review of methods for assessment of antibody purity, see, e.g. Flatman et al., J. Chromatogr. B 848:79-87 (2007).

Preferred antibodies of the invention are essentially neutralizing antibodies, i.e., they are able to at least partially inhibit an activity of PLA2-GIB.

sPLA2-GIB catalyzes the hydrolysis of the sn-2 fatty acyl bond of phospholipids to release free fatty acids and lysophospholipids. Particular antibodies of the invention inhibit an enzymatic activity of sPLA2-GIB, such as the hydrolysis of the sn-2 fatty acyl bond of phospholipids. Methods for testing such a property are disclosed in detail in the experimental section. Particular antibodies for use in the invention inhibit binding of sPLA2-GIB to a substrate thereof. Further particular antibodies for use in the invention inhibit sPLA2-GIB-mediated inhibition of IL-7-induced phospho-STAT5 nuclear translocation in CD4 T cells. Methods for testing such a property are disclosed in detail in the experimental section.

The neutralizing activity of the antibody or derivative can be determined in vitro or in vivo using e.g., binding or biological assays, such as tests as described in the experimental section. Inhibition/neutralization may be complete or partial. In particular, the antibodies may inhibit 10% or more of the tested activity, preferably 20% or more, 30% or more, 40% or more, 50% or more.

In preferred embodiments, the antibodies are IgG, e.g., gG1, IgG2, IgG3 or IgG4.

The antibodies or derivatives may be isolated and preserved using conventional methods and media. They may be lyophilized. They may also be frozen.

### Nucleic acids, vectors and host cells encoding recombinant antibodies

In another aspect, the PLA2-GIB inhibitor is or comprises or consists of a nucleic acid molecule encoding an anti-PLA2-GIB antibody, or a light or heavy chain thereof, or a variable domain thereof, or a nucleic acid complementary to said encoding sequence.

The nucleic acid can be DNA (cDNA or gDNA), RNA, or a mixture thereof. It can be in single stranded form or in duplex form or a mixture of the two. It can comprise modified nucleotides, comprising for example a modified bond, a modified purine or pyrimidine base, or a modified sugar. It can be prepared by any method known to one skilled in the art, including chemical synthesis, recombination, and mutagenesis. The nucleic acid according to the invention may be deduced from the sequence of the antibody according to the invention and codon usage may be adapted according to the host cell in which the nucleic acid shall be transcribed. These steps may be carried out according to methods well known to one of skill in the art and some of which are described in the reference manual Sambrook *et al.* (Sambrook J, Russell D (2001) Molecular cloning: a laboratory manual, Third Edition Cold Spring Harbor).

The nucleic acid may encode an amino acid sequence comprising the light chain and/or an amino acid sequence comprising the heavy chain of the antibody, or may be complementary to such encoding sequence.

Specific examples of such nucleic acid sequences include the sequences provided as SEQ ID NOs: 9-12.

The present invention further provides a vector comprising a nucleic acid of the invention. Optionally, the vector may comprise several nucleic acids of the invention. In particular, the vector may comprise a nucleic acid of the invention operably linked to a regulatory region, i.e. a region comprising one or more control sequences. Optionally, the vector may comprise several nucleic acids of the invention operably linked to several regulatory regions.

The term "control sequences" means nucleic acid sequences necessary for expression of a coding region. Control sequences may be endogenous or heterologous. Well-known control sequences and currently used by the person skilled in the art will be preferred. Such control sequences include, but are not limited to, promoter, signal peptide sequence and transcription terminator.

The term "operably linked" means a configuration in which a control sequence is placed at an appropriate position relative to a coding sequence, in such a way that the control sequence directs expression of the coding region.

The present invention further relates to the use of a nucleic acid or vector according to the invention to transform, transfect or transduce a host cell.

The present invention also provides a host cell comprising one or several nucleic acids of the invention and/or one or several vectors of the invention.

The term "host cell" also encompasses any progeny of a parent host cell that is not identical to the parent host cell due to mutations that occur during replication.

Suitable host cells for cloning or expression of antibody-encoding vectors include prokaryotic or eukaryotic cells such as bacteria, yeasts, insect cells, mammalian cells, etc.

### Inhibitory Nucleic acids

In another embodiment, the PLA2-GIB inhibitor is an inhibitory nucleic acid, i.e., any nucleic acid molecule which inhibits PLA2-GIB gene or protein expression. Preferred inhibitory nucleic acids include antisense nucleic acids, short interfering RNAs (siRNAs), small hairpin RNAs (shRNA), microRNAs, aptamers, or ribozymes. In a particular embodiment, the inhibitory nucleic acid is a small interfering RNA that prevents translation of PLA2-GIB mRNA. In another particular embodiment, the inhibitory nucleic acid is an antisense oligonucleotide that prevents translation of PLA2-GIB mRNA. In another particular embodiment, the inhibitory nucleic acid is a small hairpin RNA that prevents translation of PLA2-GIB mRNA.

siRNA comprise a sense nucleic acid sequence and an anti-sense nucleic acid sequence of the polynucleotide of interest. siRNA are constructed such that a single transcript (double stranded RNA) have both the sense and complementary antisense sequences from the target gene. The nucleotide sequence of siRNAs may be designed using an siRNA design computer program available from, for example, the Ambion website on the world wide web.

In some embodiments, the length of the antisense oligonucleotide or siRNAs is less than or equal to 10 nucleotides. In some embodiments, the length of the antisense oligonucleotides and siRNAs is as long as the naturally occurring transcript. In some embodiments, the antisense oligonucleotides and siRNAs have 18-30 nucleotides. In some embodiments, the antisense oligonucleotides and siRNAs are less than 25 nucleotides in length.

Preferred inhibitory nucleic acid molecules comprise a domain having a nucleotide sequence that is perfectly complementary to a region of a PLA2-GIB gene or RNA. Such a domain contains typically from 4 to 20 nucleotides, allowing specific hybridization and optimal inhibition the the gene transcription or RNA translation. The sequence of the inhibitory nucleic acids may be derived directly from the sequence of a gene encoding PLA2-GIB, such as SEQ ID NO: 2. Alternatively, or in addition, inhibitory nucleic acids may hybridize to a regulatory element in a PLA2-GIB gene or RNA, such as a promoter, a splicing site, etc., and prevent effective regulation thereof.

Specific examples of inhibitory nucleic acid molecules of the present invention include isolated single strand nucleic acid molecules consisting of from 10 to 50 consecutive nucleotides of a sequence encoding SEQ ID NO: 1. Specific examples of inhibitory nucleic acid molecules of the invention are antisense nucleic acids consisting of the following nucleoitide sequence or the perfectly complementary strand thereof:
ATGAAACTCCTTGTGCTAG (SEQ ID NO: 13)
ACAGCGGCATCAGC (SEQ ID NO: 14)
TTCCGCAAAATGATCAA (SEQ ID NO: 15)
CCCGGGGAGTGACCCC (SEQ ID NO: 16)
TACGGCTGCTACTGTGGCTT (SEQ ID NO: 17)
GACACATGACAACTGCTACGACC (SEQ ID NO: 18)
ACCCACACCTATTCATACTCGT (SEQ ID NO: 19)
ATCACCTGTAGCAGCA (SEQ ID NO: 20)
AGCTCCATATAACAAGGCA (SEQ ID NO: 21)
CAAGAAGTATTGTCAGAG (SEQ ID NO: 22)

### Peptides and Small Drugs

In an alternative embodiment, the PLA2-GIB inhibitor is a peptide or small drug that inhibits the activity of PLA2-GIB. The peptide or small drug is typically a molecule that selectively binds PLA2-GIB, or a substrate of PLA2-GIB, or a co-factor of PLA2-GIB, or a degradation product or metabolite of PLA2-GIB pathway.

Peptides preferably contain from 3 to 20 amino acid residues, and their sequence may be identical to a domain of PLA2-GIB (bait peptide) or to a domain of a PLA2-GIB substrate, co-factor, degradation product or metabolite. Preferred peptides of the invention contain from 4 to 30 consecutive amino acid residues of SEQ ID NO: 1 or 2 (or of a corresponding sequence of a natural variant of SEQ ID NO: 1 or 2). Most preferred peptides of the invention comprise from 5 to 25 consecutive amino acid residues of SEQ ID NO: 2 (or of a corresponding sequence of a natural variant of SEQ ID NO: 2) and further comprise at least one of the following amino acid residues of SEQ ID NO: 2 (or of a corresponding sequence of a natural variant of SEQ ID NO: 2): amino acid 3, amino acid 6, amino acid 7, amino acid 10, amino acid 70, amino acid 121, amino acid 50, amino acid 52, amino acid 54, amino acid 71, amino acid 75, amino acid 77, amino acid 79, amino acid 80, or a combination thereof. Specific examples of peptides of the invention are peptides of less than 25 amino acids comprising anyone of the following sequences:
NNYGCY (SEQ ID NO: 23)
CYCGLG (SEQ ID NO: 24)
YNNYGCYCGLGGSG (SEQ ID NO: 25)
FLEYNNYGCYCGLGGSGTPV (SEQ ID NO: 26)
QTHDN (SEQ ID NO: 27)
CQTHDNC (SEQ ID NO: 28)
ECEAFICNC (SEQ ID NO: 29)
DRNAAI (SEQ ID NO: 30)
DRNAAICFSKAPYNKAHKNL (SEQ ID NO: 31)

Other peptides for use in the invention include a pentapeptide as disclosed in WO2017/060405, incorporated therein by reference. In a specific embodiment, the compound is a cyclic peptide selected from FLSYK (SEQ ID NO: 32), FLSYR (SEQ ID NO: 33) and (2NapA)LS(2NapA)R (SEQ ID NO: 34).

The peptides of the invention can comprise peptide, non-peptide and/or modified peptide bonds. In a particular embodiment, the peptides comprise at least one peptidomimetic bond selected from intercalation of a methylene (-CH₂-) or phosphate (-PO₂-) group, secondary amine (-NH-) or oxygen (-O-), alpha-azapeptides, alpha-alkylpeptides, N-alkylpeptides, phosphonamidates, depsipeptides, hydroxymethylenes, hydroxyethylenes, dihydroxyethylenes, hydroxyethylamines, retro-inverso peptides, methyleneoxy, cetomethylene, esters, phosphinates, phosphinics, or phosphonamides. Also, the peptides may comprise a protected N-ter and/or C-ter function, for example, by acylation, and/or amidation and/or esterification.

The peptides of the invention may be produced by techniques known per se in the art such as chemical, biological, and/or genetic synthesis.

Each of these peptides, in isolated form, represents a particular object of the present invention.

Preferred small drugs are hydrocarbon compounds that selectively bind PLA2-GIB.

Examples of small drugs include indole compounds, such as those disclosed in WO2017/037041, incorporated therein by reference. In a particular embodiment, the compound is 3-(2-amino-1,2-dioxoethyl)-2-ethyl-1-(phenylmethyl)-1H-indol-4-yl)oxy)acetic acid or a pharmaceutically acceptable salt, hydrate, or prodrug thereof, such as a sodium salt thereof (Varespladib).

Small drugs and peptides are preferably obtainable by a method comprising: (i) contacting a test compound with PLA2-GIB or a fragment thereof, (ii) selecting a test compound which binds PLA2-GIB or said fragment thereof, and (iii) selecting a compound of (ii) which inhibits an activity of PLA2-GIB. Such a method represents a particular object of the invention.

Small drugs and peptides are also obtainable by a method comprising: (i) contacting a test compound with a PLA2-GIB substrate, co-factor, or degradation product, or a fragment thereof, (ii) selecting a test compound which binds to said PLA2-GIB substrate, co-factor, or degradation product, or a fragment thereof, and (iii) selecting a compound of (ii) which inhibits an activity of PLA2-GIB. Such a method represents a particular object of the invention.

### Vaccination

In an alternative (or cumulative) embodiment, the PLA2-GIB inhibitor is a PLA2-GIB antigen. As a result of a vaccination or immunization of the subject with said antigen, the subject produces antibodies (or cells) which inhibit PLA2-GIB. In particular, injection(s) of a PLA2-GIB antigen (e.g., an immunogenic PLA2-GIB essentially devoid of biological activity) can generate antibodies in the treated subject. These antibodies will protect against an excess of PLA2-GIB expression and can be used along as immunotherapy or a vaccine prophylaxy.

An object of the invention thus resides in a method of treating a solid cancer in a subject having solid cancer, comprising administering to the subject a PLA2-GIB antigen.

A further object of the invention relates to a PLA2-GIB antigen for use to treat a solid cancer in a subject in need thereof.

In a particular embodiment, the PLA2-GIB antigen is an inactivated immunogenic molecule that induces an immune response against PLA2-GIB in a subject. Inactivation may be obtained e.g., by chemically or physically altering PLA2-GIB or by mutating or truncating the protein, or both; and immunogenicity may be obtained as a result of the inactivation and/or by further conjugating the protein to a suitable carrier or hapten, such as KLH, HSA, polylysine, a viral anatoxin, or the like, and/or by polymerization, or the like. The antigen may thus be chemically or physically modified, e.g., to improve its immunogenicity.

In a preferred embodiment, the PLA2-GIB antigen comprises PLA2-GIB or an epitope-containing fragment or mimotope thereof.

In a particular embodiment, the PLA2-GIB antigen comprises a full length PLA2-GIB protein. In a further particular embodiment, the PLA2-GIB antigen comprises a protein comprising SEQ ID NO: 2, or a sequence having at least 90% identity to SEQ ID NO: 2.

In an alternative embodiment, the PLA2-GIB antigen comprises a fragment of a PLA2-GIB protein comprising at least 6 consecutive amino acid residues and containing an immunogenic epitope, or a mimotope thereof. In a preferred embodiment, the PLA2-GIB antigen comprises at least from 6 to 20 amino acid residues. Preferred peptides of the invention contain from 4 to 30 consecutive amino acid residues of SEQ ID NO: 2 (or of a corresponding sequence of a natural variant of SEQ ID NO: 2). Most preferred peptides of the invention comprise from 5 to 25 consecutive amino acid residues of SEQ ID NO: 2 (or of a corresponding sequence of a natural variant of SEQ ID NO: 2) and further comprise at least one of the following amino acid residues of SEQ ID NO: 2 (or of a corresponding sequence of a natural variant of SEQ ID NO: 2): amino acid 3, 6, 7, 10, 70, 121, 50, 52, 54, 71, 75, 77, 79, 80, or a combination thereof. Specific examples of peptides of the invention are peptides of less than 50 amino acids comprising anyone of the following sequences:
NNYGCY (SEQ ID NO: 23)
CYCGLG (SEQ ID NO: 24)
YNNYGCYCGLGGSG (SEQ ID NO: 25)
FLEYNNYGCYCGLGGSGTPV (SEQ ID NO: 26)
QTHDN (SEQ ID NO: 27)
CQTHDNC (SEQ ID NO: 28)
ECEAFICNC (SEQ ID NO: 29)
DRNAAI (SEQ ID NO: 30)
DRNAAICFSKAPYNKAHKNL (SEQ ID NO: 31)

The PLA2-GIB antigen may be in various forms such as in free form, polymerized, chemically or physically modified, and/or coupled (i.e., linked) to a carrier molecule. Coupling to a carrier may increase the immunogenicity and (further) suppress the biological activity of the PLA2-GIB polypeptide. In this regard, the carrier molecule may be any carrier molecule or protein conventionally used in immunology such as for instance KLH (Keyhole limpet hemocyanin), ovalbumin, bovine serum albumin (BSA), a viral or bacterial anatoxin such as toxoid tetanos, toxoid diphteric B cholera toxin, mutants thereof such as diphtheria toxin CRM 197, an outer membrane vesicle protein, a polylysine molecule, or a virus like particle (VLP). In a preferred embodiment, the carrier is KLH or CRM197 or a VLP.

Coupling of PLA2-GIB to a carrier may be performed by covalent chemistry using linking chemical groups or reactions, such as for instance glutaraldehyde, biotin, etc. Preferably, the conjugate or the PLA2-GIB protein or fragment or mimotope is submitted to treatment with formaldehyde in order to complete inactivation of PLA2-GIB.

In a particular embodiment, the PLA2-GIB antigen comprises a full length PLA2-GIB protein, optionally coupled to a carrier protein. In a preferred embodiment, the PLA2-GIB antigen comprises a protein comprising SEQ ID NO: 2, or a sequence having at least 90% identity to SEQ ID NO: 2, coupled to a carrier protein.

In another particular embodiment, the PLA2-GIB antigen comprises an immunogenic peptide or mimotope of PLA2-GIB, optionally coupled to a carrier protein. In a more preferred embodiment, the PLA2-GIB antigen comprises a polypeptide of at least 10 amino acids long comprising at least one of the following amino acid residues of SEQ ID NO: 2 (or of a corresponding sequence of a natural variant of SEQ ID NO: 2): amino acid 70, amino acid 121, amino acid 50, amino acid 52, amino acid 54, amino acid 71, or a combination thereof, optionally coupled to a carrier molecule.

The immunogenicity of the PLA2-GIB antigen may be tested by various methods, such as by immunization of a non-human animal grafted with human immune cells, followed by verification of the presence of antibodies, or by sandwich ELISA using human or humanized antibodies. The lack of biological activity may be verified by any of the activity tests described in the application. In a preferred embodiment, the PLA2-GIB antigen has less than 20%, more preferably less than 15%, 10%, 5% or even 1% of the activity of a wild-type PLA2-GIB protein in an in vitro method of (i) induction of formation of membrane microdomains (MMD) in CD4 T cells or (ii) in rendering CD4 T cells refractory to IL-2 signaling or refractory to IL-7 signaling.

Such molecules and conjugates and vaccines represent potent agents for use to immunize subjects, thereby causing a sustained PLA2-GIB inhibition. Upon repetition, such methods can be used to cause a permanent PLA2-GIB inhibition.

Further aspects and advantages of the invention are disclosed in the following experimental section, which shall be considered as illustrative.

### EXAMPLES

### A. Materials and Methods

### A1. PLA2GIB sandwich ELISAs

Sandwich ELISAs specific to proPLA2-GIB, PLA2GIB or both forms were performed using mAbs previously generated. For the detection of proPLA2GIB, the #8G11 mAb was used as a capture antibody and the #1C11 mAb was used as a revelation mAb. For the detection of PLA2GIB, the #14G9 mAb was used as a capture antibody and the #1C11 mAb was used as a revelation mAb. For the detection of both forms, the #7A10 mAb was used as a capture antibody and the #1C11 mAb was used as a revelation mAb. Typical assay conditions were as follows: #8G11- or #14G9-coated microplate was incubated for one hour with recombinant human proPLA2GIB or PLA2GIB standards (varying concentrations of protein in assay buffer) to generate a calibration curve. EDTA plasma samples were diluted (5- to 50-fold) in dilution buffer to their respective wells and the ELISA plate was incubated for 1 h at room temperature. After aspiration, the wells were washed 3 times with PBS containing Tween 20 0.05%, and the biotinylated #1C11 conjugate was added to the wells for 30 min at room temperature. Following washing with PBS containing 0.05% Tween 20, the binding of mAb was detected by treatment with a Streptavidin-HRP conjugate for 15 min at room temperature. TMB was added, reaction was stopped and absorbance at 450 nm was determined on a microplate reader. Signals were analyzed with a 4-parameter fit analysis using Prism™ software (Graph Pad).

### A2. IL7-induced phospho-STAT5 nuclear translocation

Human CD4 were purified using the RosetteSep isolation kit (Stemcell Technologies, #15062). CD4 T-cells were resuspended at 10⁶ cells/ml in RPMI 1640 medium (Lonza, Verviers, Belgium) supplemented with 5% foetal bovine serum (FBS), 50 mM HEPES pH 7.4, glutamine, penicillin, streptomycin and fungizone (complete medium) and equilibrated at least 2 h at 37°C in a 5% CO2 humidified atmosphere.

pSTAT5 nuclear translocation was followed using confocal microscopy. After equilibration, immobilized cells were plated on polylysine-coated glass slides in presence of plasma (final concentration 1% or 3%) before activation for 15 min with 2 nM IL-7.

For the PLA2GIB neutralization experiments, 10 µg (final concentration) of #14G9 mAb were first incubated for 25 min at room temperature, followed by 5 min at 37°C, together with plasma (1 or 3% dilution v/v in PBS). The equilibrated mixture was then added to CD4 T cells isolated from healthy donors during 30 min at 37°C. The cells were then activated for 15 min with 2 nM IL-7.

The cells were fixed in 4% PFA for 15 min at 37°C then 15 min at RT and permeabilized in 90% methanol/water solution at -20°C. pSTAT5 was then revealed by staining with rabbit anti-pSTAT5 (CST, #9359) labelled with donkey anti-rabbit-AlexaFluor 555 (Life Technologies, #A31572). For human CD4 T lymphocytes, cells were stained with mouse anti-human CD4 (eBioscience, #14-0042-82) labelled with goat anti-mouse-AlexaFluor 488 (Life Technologies, #A11029). For mice CD4 T lymphocytes cells were stained with rat anti-mouse CD4 (eBioscience, #14-0042-85) labelled with chicken anti-rat-AlexaFluor 488 (Life Technologies, #A21470). Images were acquired above the diffraction limit on an inverted laser scanning confocal microscope (LSM700, Zeiss) with a water-immersion apochromatic 40x/1.2 NA objective lens (Zeiss) or an oil-immersion plan-apochromatic 63x/1.4 NA objective lens (Zeiss). Images were acquired and analyzed with the ZEN software (Zeiss).

### A3. IL2-induced phospho-STAT5 nuclear translocation

The same protocol as in A2 was used, except that 2 nM IL-2 was used instead of IL-7.

### B. Results

### B1. Detection of proPLAGIB, PLA2GIB, or both forms by ELISA in plasma from PDAC patients

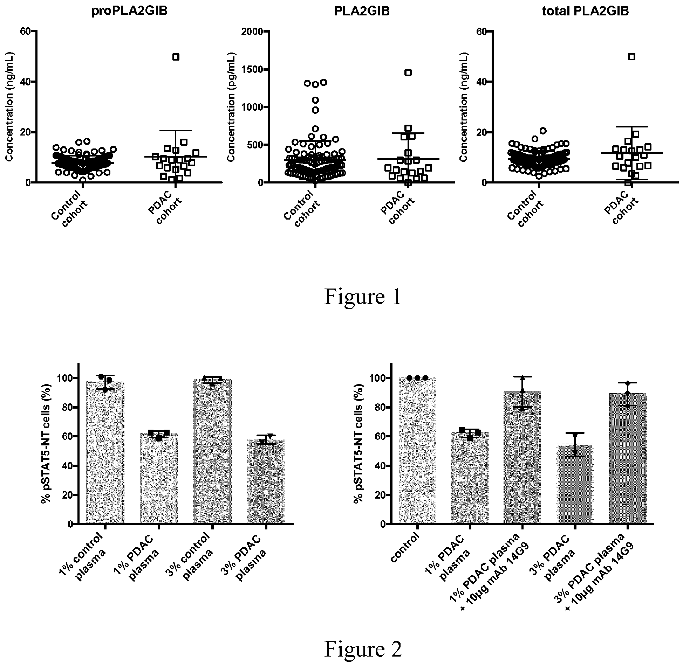

ELISA sandwich methods were used to examine the proPLA2GIB and PLA2GIB distribution in plasma from patients with pancreatic ductal adenocarcinoma ("PDAC"). The results are presented Figure 1.

In the control population (n=99 healthy blood donors), the mean (SD) proPLA2GIB level was 7.8 (2.8) ng/mL and the median was 7.5 ng/mL. The mean (SD) PLA2GIB was 287 (243) pg/mL and the median was 170 pg/mL. The mean (SD) value for total PLA2GIB was 9.3 (3.2) ng/mL and the median was 9.1 ng/mL.

In the PDAC population (n=19), the mean (SD) proPLA2GIB level was 10.1 (10.4) ng/mL and the median was 8.9 ng/mL; the mean (SD) PLA2GIB level was 310 (345) pg/mL and the median was 194 pg/mL; and the mean (SD) total PLA2GIB level was 11.7 (10.5) ng/mL and the median was 10.4 ng/mL.

In this experiment, there was no statistically significant differences in PLA2GIB levels in plasma between control and PDAC cohorts.

### B2. In vitro effect of the PDAC plasma on in the activity of human CD4 T cells.

We tested the capacity of the plasma from PDAC patients to modulate IL-7 response of CD4 T cells by measuring the phospho-STAT5 nuclear translocation (pSTAT5 NT).

As shown in Figure 2A, we observed an inhibitory effect of PDAC plasma on CD4 T cell IL-7 response at 1% and 3% dilution. This results demonstrates that, in cancer patients, the tumor microenvironment or plasma provides immune modulation, e.g. inhibition.

### B3. Effect of a PLA2-GIB inhibitor on the PDAC plasma-induced inactivation of human CD4 T cells.

The ability of the a neutralizing anti-PLA2GIB mAb (clone 14G9) to counterbalance the inhibitory effect of plasma isolated from PDAC patients on the IL-7 induced phospho-STAT5 nuclear translocation in CD4 T cells was tested by incubating plasma samples from PDAC patients with the #14G9 mAb.

As depicted in Figure 2B, the #14G9 mAb restored effective IL-7-induced nuclear translocation of phosphoSTAT5 in human CD4 T cells of healthy donors exposed to plasma samples from PDAC patients. A PLA2-GIB inhibitor is thus able to improve cancer subjects. This finding indicates that cancers contain a PLA2-GIB cofactor which renders T cells sensitive to inactivation by PLA2-GIB.

### B4. In vitro effect of the PDAC plasma on IL2-induced phospho-STAT5 nuclear translocation in human CD4 T cells.

We tested the capacity of the plasma from PDAC patients to modulate IL-2 response of CD4 T cells by measuring the phospho-STAT5 nuclear translocation (pSTAT5 NT).

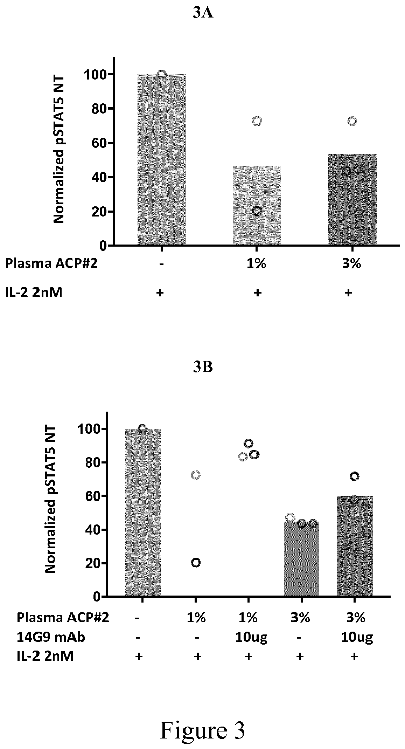

As shown in Figure 3A, we observed an inhibitory effect of PDAC plasma on CD4 T cell IL-2 response at 1% and 3% dilution. This results demonstrates that, in cancer patients, the tumor microenvironment or plasma provides immune inhibition.

### B5. Effect of a PLA2-GIB inhibitor on the PDAC plasma-induced inactivation of human CD4 T cells.

The ability of the a neutralizing anti-PLA2GIB mAb (clone 14G9) to counterbalance the inhibitory effect of plasma isolated from PDAC patients on the IL2-induced phospho-STAT5 nuclear translocation in CD4 T cells was tested by incubating plasma samples from PDAC patients with the #14G9 mAb.

As depicted in Figure 3B, the #14G9 mAb restored effective IL2-induced nuclear translocation of phosphoSTAT5 in human CD4 T cells of healthy donors exposed to plasma samples from PDAC patients. A PLA2-GIB inhibitor is thus able to improve cancer subjects. This finding indicates that cancers contain a PLA2-GIB cofactor which renders T cells sensitive to inactivation by PLA2-GIB.

### B6. In vitro effect of the plasma from cancer patients on human CD4 T cells.

The effect of the plasma from patients with lung cancer and duodenum cancer is tested on human T cells, as disclosed in B2 above. More particularly, a dilution of said plasma is prepared (1%) and such sample is contacted to CD4 T cells from a healthy donor. The ability of such samples to modulate the activity of CD4 T cells is analyzed by measuring the phospho-STAT5 nuclear translocation (pSTAT5 NT) in the presence of IL7.

An inhibitory effect of said plasma samples can show that in lung cancer patients, the tumor microenvironment or plasma provides immune inhibition which can be restored according to the present invention.

## Claims

1. A compound which inhibits PLA2-GIB for use for treating cancer or neoplasia in a subject in need thereof.

2. The compound for use according to claim 1, wherein the compound is an antibody or a fragment or derivative thereof which binds PLA2-GIB.

3. The compound for use according to claim 2, wherein the compound is a monoclonal antibody.

4. The compound for use according to claim 3, wherein the monoclonal antibody is human or humanized.

5. The compound for use according to claim 1, wherein the compound is 3-(2-amino-1,2-dioxoethyl)-2-ethyl-1-(phenylmethyl)-1H-indol-4-yl)oxy)acetic acid or a pharmaceutically acceptable salt, hydrate, or prodrug thereof, such as a sodium salt thereof.

6. The compound for use according to claim 1, wherein the compound is a vaccine against PLA2-GIB.

7. The compound for use according to claim 1, wherein the compound is a cyclic peptide selected from FLSYK, FLSYR and (2NapA)LS(2NapA)R.

8. The compound for use according to anyone of claims 1 to 7, wherein the compound is administered in combination with a further anticancer agent, vaccine, or treatment.

9. The compound for use according to claim 8, wherein the compound is administered in combination with chemotherapy or hormonotherapy.

10. The compound for use according to claim 8, wherein the compound is administered in combination with radiotherapy, ultrasound therapy or nanoparticle therapy.

11. The compound for use according to claim 8, wherein the compound is administered in combination with check-point inhibitors, immunotherapy or anti-cancer vaccines.

12. The compound for use according to claim 8, wherein the compound is administered in combination with an inhibitor of a cofactor of PLA2-GIB.

13. The compound for use according to claim 12, wherein the inhibitor is an antagonist of a PLA2-GIB cofactor, or a cytostatic or cytotoxic agent or a vaccine against a PLA2-GIB cofactor or against a prokaryotic or eukaryotic cell or virus expressing a PLA2-GIB cofactor.

14. The compound for use according to anyone of claims 1 to 13, wherein the compound is administered prior to, during or after surgery.

15. The compound for use according to anyone of claims 1 to 14, wherein the compound is administered repeatedly.

16. The compound for use according to anyone of the preceding claims, wherein the cancer is a solid cancer.

17. The compound for use according to anyone of claims 1 to 16, wherein a PLA2-GIB cofactor or a prokaryotic or eukaryotic cell or virus expressing PLA2-GIB cofactor is present in said subject.

18. The compound for use according to anyone of claims 1 to 17, wherein PLA2-GIB or a PLA2-GIB cofactor is present in the cancer microenvironment or blood.

19. The compound for use according to anyone of claims 1 to 18, wherein the subject has a PLA2GIB-related CD4 T cell deficiency.

20. The compound for use according to claim 16, wherein the cancer is selected from pancreatic cancer, melanoma, lung, oesophageal or pharyngeal cancer, retinoblastoma, liver, breast, ovary, renal, gastric, duodenum, uterine, cervical, thyroid, bladder, prostate, bone, brain or colorectal cancer.

21. The compound for use according to claim 20, wherein the cancer is pancreatic cancer.

22. The compound for use according to claim 21, wherein the cancer is selected from pancreatic adenocarcinoma, neuroendocrine tumor, intraductal papillary-mucinous neoplasama, mucinous cystic neoplasm, and serious cystic neoplasm.

23. The compound for use according to claim 1, wherein the cancer is inducing gastrointestinal and metabolic pathologies.

24. The compound for use according to anyone of claims 1 to 23, for preventing cancer or reducing the rate of cancer occurrence.

25. The compound for use according to anyone of claims 1 to 23, for reducing the rate of cancer progression.

26. The compound for use according to anyone of claims 1 to 23, for reducing or preventing or treating cancer metastasis.

27. The compound for use according to anyone of claims 1 to 23, for killing cancer cells.

28. The compound for use according to anyone of claims 1 to 23, for treating risk factors for cancer, particularly oro-gastro-intestinal inflammations or infections, such as pancreatitis.

29. The compound for use according to anyone of claims 1 to 28, wherein the subject is a human.

30. The compound for use according to anyone of claims 1 to 29, wherein the level of PLA2-GIB or PLA2-GIB cofactor in the tumor or in body fluids is measured to guide therapeutic regimen.
